(19) Europäisches Patentamt
European Patent Office
Office européen des brevets

(11) Veröffentlichungsnummer: **0 065 226**
**A2**

(12) # EUROPÄISCHE PATENTANMELDUNG

(21) Anmeldenummer: **82103911.2**

(22) Anmeldetag: **06.05.82**

(51) Int. Cl.³: **C 07 D 239/42,** C 07 D 249/14, C 07 D 285/08, C 07 D 285/12, C 07 D 213/76, C 07 D 231/52, C 07 D 257/06, C 07 D 279/06, C 07 D 271/10, C 07 D 271/06, C 07 D 231/38

(30) Priorität: **07.05.81 DE 3118127**

(43) Veröffentlichungstag der Anmeldung: **24.11.82 Patentblatt 82/47**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Busse, Wolf-Dieter, Dr., Pahlkestrasse 65, D-5600 Wuppertal 1 (DE)**
Erfinder: **Krauthausen, Edmund, Dr., Franz-Grillparzer-Ring 4, D-5000 Köln 71 (DE)**
Erfinder: **Mardin, Mithat, Dr., Bergerheide 39, D-5600 Wuppertal 1 (DE)**

(54) **Neue Sulfenamide, Verfahren zu ihrer Herstellung, ihre Verwendung in Arzneimitteln und deren Herstellung.**

(57) Die vorliegende Erfindung betrifft neue Sulfenamide der allgemeinen Formel I

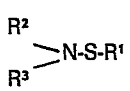

(I)

worin R¹, R² und R³ die in der Beschreibung angegebene Bedeutung haben, ein Verfahren zu ihrer Herstellung, ihre Verwendung als Arzneimittel, insbesondere ihre Verwendung als Lipoxygenasehemmer, diese enthaltende Arzneimittel und deren Herstellung.

EP 0 065 226 A2

0065226

BAYER AKTIENGESELLSCHAFT 5090 Leverkusen, Bayerwerk

Zentralbereich
Patente, Marken und Lizenzen KS-by-c
Ia(Pha)

Neue Sulfenamide, Verfahren zu ihrer Herstellung, ihre Verwendung in Arzneimitteln und deren Herstellung

Die vorliegende Erfindung betrifft neue Sulfenamide, ein Verfahren zu ihrer Herstellung, ihre Verwendung als Arzneimittel, insbesondere ihre Verwendung als Lipoxygenasehemmer, diese enthaltende Arzneimittel und deren Herstellung.

Es ist bekannt, daß die durch das Enzym Lipoxygenase gebildeten Metaboliten der Arachidonsäure an der Entstehung von entzündlichen und allergischen Prozessen beteiligt sind (vgl. E.J. Goetzl, Immunology 40, 709 (1980); Ford-Hutchinson et al., J. Pharm. Pharmacol. 32, 517 (1980) und Nature 286, 264 (1980) und Samuelsson, Trends in Pharmacol. Sci., Mai 1980, 227; Borgeat et al., J. Med. Chem. 24, 121 (1981)).

Bekannte Lipoxygenasehemmer wie Nordihydroguaretsäure, 3-Amino-1-(3-trifluormethylphenyl)-pyrazolin, Phenidon und 5,8,11,14-Eikosatetrainsäure sind entweder gleichzeitig als Cyclooxygenasehemmer oder erst bei sehr hohen Konzentrationen wirksam. Die Hemmung des Enzyms Cyclooxygenase des Arachidonsäuremetabolismus führte zu einer globalen Prostaglandinsynthesehemmung und zu einer Stimulation des Lipoxygenaseweges, was eine Gastrotoxizität bzw. proinflammatorische und asthmatische Wirkungen verursacht (vgl. Yen und Kreutner, Agents and Actions, 10, 274 (1980) und Blackwell und Flower, Prostaglandins 16, 417 (1978); und vgl. ebenso Brune et al., J. Pharm. Pharmacol. 33, 127-128 (1981); Higgs et al., Eur. Pharmacol. 66, 81-86 (1980) und Piper et al, Prostaglandins 19, 371 (1980)). Es besteht ein dringendes Bedürfnis nach Verbindungen, die diese unerwünschten Nebenwirkungen nicht besitzen.

Überraschenderweise hemmen die erfindungsgemäßen neuen Sulfenamide die Lipoxygenase sehr spezifisch bereits in solchen Konzentrationen, bei denen die Cyclooxygenase nicht beeinflußt wird. Bei Kenntnis des Standes der Technik konnte diese sehr starke und spezifische Wirkung der Sulfenamide nicht erwartet werden. Die erfindungsgemäßen, Lipoxygenase-hemmenden Sulfenamide sind somit als Arzneimittel bei der Behandlung von entzündlichen und allergischen Prozessen einsetzbar. Sie können insbesondere als Antiphlogistika, Antirheumatika, Antiatherosklerotika, Antiasthmatika, Antiallergika, Antimetastatika und Gastroprotektiva Verwendung finden.

Die erfindungsgemäßen Sulfenamide entsprechen der allgemeinen Formel I

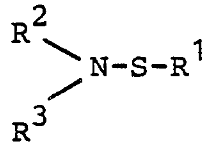

(I)

worin

$R^1$ für Alkyl mit 1 bis 18 C-Atomen, Alkenyl oder Alkinyl mit 2 bis 12 C-Atomen, Aralkyl mit 7 bis 11 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkoxy, Alkyl, Aralkyl, Cycloalkyl, Aryl, Aryloxy, Arylthio, Alkylthio, Carboxy, Carbalkoxy, Cyano, Carbamoyl, Sulfonyl, Halogenalkyl, Halogenalkoxy, Halogen, Amino oder substituiertes Amino substituiert sind oder $R^1$ für den Rest

$$-Q-S-NR^2R^{3'} \qquad (Ia)$$

steht,

wobei

$R^2$ die unten angegebene Bedeutung hat und

$R^{3'}$ die unter $R^3$ angegebene Bedeutung hat außer einem Rest der Formel (Ia),

Q für Alkylen mit 1 bis 12 C-Atomen, wobei der Alkylenrest durch Sauerstoff, Schwefel oder Stickstoff ein- oder mehrfach unterbrochen sein kann, Cycloalkylen mit 5 bis 12 C-Atomen, Arylen mit 6 bis 10 C-Atomen, Alkylen-Cycloalkylen-Alkylen oder Alkylen-Arylen-Alkylen steht,

mit der Einschränkung, daß $R^1$ nicht für Trichlormethyl steht,

$R^2$ einen heterocyclischen Rest mit 5 bis 8 Ringgliedern, welcher 1 bis 4 Heteroatome aus der Gruppe Sauerstoff, Schwefel oder Stickstoff enthält, wobei dieser heterocyclische Rest gegebenenfalls mit einem Arylrest annelliert ist und bis zu 5 gleiche oder verschiedene Substituenten aus der Gruppe $R^{1'}$, worin

$R^{1'}$ einer der Reste der Definition von $R^1$, jedoch nicht den Rest der Formel (Ia), bedeutet;

Halogen, Alkylthio, Cycloalkylthio, Aralkylthio, Arylthio, Alkoxy, Aryloxy, Cyano, Nitro, Cycloalkoxy, Aralkoxy oder $-CO-R^4$, worin

$R^4$ für Wasserstoff, Alkyl, Cycloalkyl, Alkenyl, Alkinyl, Aralkyl, Aryl, Alkoxy, Aralkoxy, Aryloxy, Alkylthio, Arylthio oder $-NR^{3''}R^5$ steht, worin

$R^{3''}$ die unter $R^{3'}$ angegebene Bedeutung hat, jedoch nicht den Rest $R^2$ und

$R^5$ Wasserstoff oder einen der unter $R^{1'}$ genannten Reste bedeutet,

oder einen Rest der allgemeinen Formel

$$-W-CO-NR^{3''}R^{5'} \qquad \text{(Ib)}$$

bedeutet,

wobei

W für eine direkte Bindung, eine Disulfidbrücke oder für eines der oben unter Q genannten Brückenglieder steht, und

$R^{5'}$ die in der Definition für $R^5$ außer dem Rest der Formel (Ib) angegebenen Bedeutung besitzt, oder

$SO_2$-$R^6$, worin

$R^6$ für Fluoralkyl, einen der unter $R^4$ aufgeführten Reste oder für

$$-W-SO_2-NR^{3''}R^5 \qquad (Ic)$$

steht, wobei

$R^{3''}$, $R^5$ und W die oben angegebene Bedeutung haben,

oder

$-NR^{3''}R^5$, Trifluormethyl, Trifluormethoxy, Oxo, Thiono, Imino oder substituiertes Imino enthalten kann, wobei als Substituenten der Iminogruppe die oben unter $R^1$ genannten Reste oder ein Carbamoylrest, der am Stickstoff einen Substituenten aus der Gruppe Alkyl, Aryl oder Cycloalkyl trägt, genannt seien, mit der Einschränkung, daß $R^2$ nicht für 2-Oxazolyl, 2-Benzoxazolyl, 2-Thiazolyl, 2-Benzothiazolyl und 2-Benzimidazolyl steht, und

$R^3$ für einen der unter $R^1$ oder $R^2$ aufgezählten Reste steht oder Wasserstoff oder $-S-R^1$ bedeutet.

Bevorzugt sind Sulfenamide der allgemeinen Formel I, in welcher

$R^1$ für Alkyl mit 1 bis 18 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl mit 5 bis 7 Kohlenstoffatomen, Phenyl, Phenoxy, Phenylthio, Alkylthio mit 1 bis 4 Kohlenstoffatomen, Carboxy, Carbalkoxy mit 1 bis 4 Kohlenstoffatomen, Cyano, Trifluormethyl, Trifluormethoxy, Fluor, Chlor, Brom, Amino, Alkyl- oder Benzyl-substituiertes Amino, wobei die Alkylreste 1 bis 4 Kohlenstoffatome tragen, substituiert sind, mit der Einschränkung, daß $R^1$ nicht für Trichlormethyl steht,

$R^2$ für einen heterocyclischen Rest mit 5 bis 8 Ringgliedern steht, welcher 1 bis 4 Heteroatome aus der Gruppe Sauerstoff, Schwefel oder Stickstoff enthält, wobei dieser heterocyclische Rest gegebenenfalls mit einem Arylrest mit 6 bis 14 C-Atomen annelliert ist und bis zu 5 gleiche oder verschiedene Substituenten aus der Gruppe Fluor, Chlor, Brom, Alkylthio mit 1 bis 4 C-Atomen, Benzylthio, Phenylthio, Alkoxy mit 1 bis 4 C-Atomen, Phenoxy, Benzyloxy, einen der unter $R^1$ genannten Reste, $-NR^{3''}R^5$, $-CO-R^4$, Trifluormethyl, Trifluormethoxy, Oxo oder Thiono enthalten kann, mit der Einschränkung, daß $R^2$ nicht für 2-Oxazolyl, 2-Benzoxazolyl, 2-Thiazolyl, 2-Benzothiazolyl und 2-Benzimidazolyl steht, und

$R^3$ für einen der unter $R^1$ oder $R^2$ aufgezählten Reste steht oder Wasserstoff oder $-S-R^1$ bedeutet, wobei $R^1$ und $R^2$ die angegebene bevorzugte Bedeutung besitzen.

Besonders bevorzugt sind Sulfenamide der allgemeinen Formel (I), in welcher

$R^1$ für einen Arylrest mit 6 bis 14 C-Atomen steht, der gegebenenfalls mit bis zu 2 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl mit 5 bis 7 Kohlenstoffatomen, Phenyl, Phenoxy, Phenylthio, Alkylthio mit 1 bis 4 Kohlenstoffatomen, Carbalkoxy mit 1 bis 4 Kohlenstoffatomen, Cyano, Trifluormethyl, Trifluormethoxy, Fluor, Chlor, Brom, Alkyl- oder Benzyl-substituiertes Amino, wobei die Alkylreste 1 bis 4 Kohlenstoffatome tragen, substituiert ist,

$R^2$ für einen heterocyclischen Rest mit 5 oder 6 Ringgliedern, welcher 1 bis 4 Heteroatome aus der Gruppe Sauerstoff, Schwefel oder Stickstoff enthält, wobei dieser heterocyclische Rest gegebenenfalls benzanelliert ist und bis zu 3 gleiche oder verschiedene Substituenten aus der Gruppe Fluor, Chlor, Brom, Alkylthio mit 1 bis 4 C-Atomen, Benzylthio, Phenylthio, Alkoxy mit 1 bis 4 C-Atomen, Phenoxy, Benzyloxy, Alkyl mit 1 bis 12 C-Atomen, Benzyl, Phenyl, $-NR^{3''}R^5$, $-CO-R^4$, Trifluormethyl, Trifluormethoxy, Oxo oder Thiono enthalten kann, mit der Einschränkung, daß $R^2$ nicht für 2-Oxazolyl, 2-Benzoxazolyl, 2-Thiazolyl, 2-Benzothiazolyl und 2-Benzimidazolyl steht, und

$R^3$ für Wasserstoff oder Methyl steht.

$R^2$ kann beispielsweise für einen der folgenden Reste stehen:

1,3,4-Thiadiazol-2-yl
1,2,4-Thiadiazol-3-yl
1,2,4-Thiadiazol-5-yl
1,3,4-Oxadiazol-2-yl
1,2,4-Oxadiazol-3-yl
1,2,4-Oxadiazol-5-yl
1H-Pyrazol-3-yl
4H-Pyrazol-3-yl
1H-4,5-Dihydropyrazol-5-on-3-yl
1,3,4-Triazol-2-yl
1,2,4-Triazol-3-yl
1H-Tetrazol-5-yl
2H-Tetrazol-5-yl
Oxazolin-5-on-2-yl
Thiazolin-5-on-2-yl
Imidazolin-5-on-2-yl
Oxazolin-4-on-2-yl
Thiazolin-4-on-2-yl
Benzisoxazol-3-yl
Benzisothiazol-3-yl
1,2,3-Triazin-4-yl
1,2,4-Triazin-5-yl
2-Pyridyl
3-Pyridyl
4-Pyridyl
3H-Indol-2-yl
Indol-3-yl
1H-Indazol-3-yl
Isothiazol-3-yl
Isoxazol-3-yl
2H-Pyrrol-2-on-5-yl
2H-Pyrrol-5-yl
3H-Pyrrol-3-on-2-yl
3H-Pyrrol-2-yl
Imidazolin-4-on-2-yl
5H-Imidazol-5-on-2-yl
1,2,5-Oxadiazol-3-yl
2H-1,2,3-Triazol-4-yl
1,2,3,4-Oxatriazol-5-yl
1,2,3,5-Oxatriazol-4-yl
1,2,3,5-Thiatriazol-4-yl
1,2,3,4-Thiatriazol-5-yl
1,3,5-Triazin-2-yl
1,2,4-Triazin-6-yl
1,2,4-Triazin-3-yl
2H-Azepin-7-yl
4H-Azepin-7-yl
3H-Azepin-2-yl
1,3-Oxazepin-2-yl
1H-1,2-Diazepin-3-yl

2-Pyrimidinyl
4-Pyrimidinyl
5-Pyrimidinyl
2-Pyrazinyl
3-Pyridazinyl
4-Pyridazinyl
1,2,3,4-Tetrazin-5-yl
1,2,4,5-Tetrazin-3-yl
2H-1,3,5-Thiadiazin-6-yl
2H-1,3,5-Thiadiazin-4-yl
2H-1,3,5-Thiadiazin-2-yl
4H-1,3,5-Thiadiazin-2-yl
2H-1,3,5-Oxadiazin-2-yl
2H-1,3,5-Oxadiazin-4-yl
2H-1,3,5-Oxadiazin-6-yl
4H-1,3,5-Oxadiazin-2-yl
4H-1,3,5-Oxadiazin-4-yl
1,2,5-Oxathiazin-6-yl
1,2,4-Oxathiazin-3-yl
1,2,4-Oxathiazin-5-yl
1,2,4-Oxathiazin-6-yl
4H-1,2-Diazepin-7-yl
4H-1,2-Diazepin-3-yl
1H-1,3-Diazepin-2-yl
4H-1,3-Diazepin-2-yl
5H-1,3-Diazepin-2-yl
2H-1,4-Diazepin-3-yl
5H-1,4-Diazepin-3-yl
5H-1,4-Diazepin-2-yl
2H-1,4-Diazepin-7-yl
1,4-Thiazepin-5-yl
1,3-Thiazepin-2-yl
1,4-Oxazepin-5-yl
1,4-Thiazepin-2-yl
1,4-Thiazepin-3-yl
2-Azocinyl
3-Azocinyl
4-Azocinyl
5-Azocinyl

Die erfindungsgemäßen Verbindungen der allgemeinen Formel lassen sich herstellen, indem man Sulfenylhalogenide der allgemeinen Formel

$$R^1 - S - X \qquad (II)$$

in welcher

X für Chlor, Brom oder Jod steht und

$R^1$ die oben angegebene Bedeutung hat,

mit Aminen der allgemeinen Formel

$$Y - NR^2R^3 \quad (III)$$

in welcher

Y für Wasserstoff, Trialkylsilyl, ein Metall wie K, Na, Li, Mg, Ca, Ba, Ag, Cu, Zn, Fe, Mn, Pb, Sn oder Al oder einen Ammoniumrest steht und

$R^2$ und $R^3$ die oben angegebene Bedeutung haben,

in Gegenwart von inerten aprotischen organischen Lösungsmitteln und gegebenenfalls in Gegenwart von Wasser und von Basen bei Temperaturen zwischen -80° und 150°C unter Abspaltung von XY umsetzt.

Für den Fall, daß Y Wasserstoff bedeutet, ist es zweckmäßig, die Umsetzung in Gegenwart einer Base durchzuführen. Als Base seien vorzugsweise genannt organische Verbindungen wie Triethylamin, Tributylamin, Benzyldimethylamin, Dimethylanilin, Pyridin oder Chinolin. Die Reaktion wird vorzugsweise in Gegenwart eines aprotischen Lösungsmittels wie z.B. Hexan, Ligroin, Benzol, Toluol, Chlorbenzol, Chloroform, Tetrachlorkohlenstoff, Dimethylsulfoxid oder Dimethylformamid durchgeführt.

Für den Fall, daß Y nicht für einen Trialkylsilylrest steht, kann es zweckmäßig sein, die Herstellung in einem wäßrig-organischen Zweiphasenmedium durchzuführen. In diesem Falle können auch anorganische Basen als Halogenwasserstoff-Fänger eingesetzt werden, wie z.B. Alkali- oder Erdalkalihydroxide oder -carbonate.

Die Reaktion kann bei Temperaturen zwischen -80 und +150°C, vorzugsweise zwischen 0 und 50°C, durchgeführt werden.

Die für die Durchführung der Erfindung geeigneten Amine der allgemeinen Formel (III) sind bekannt oder können nach bekannten Methoden hergestellt werden (Literatur: z.B. A. Weissberger (Ed.), The Chemistry of Heterocyclic Compounds, Interscience, New York 1950 bis 1979; S. Coffey (Ed.), Rodd's Chemistry of Carbon Compounds, 2. Aufl., Vol. IV A-K, Elsevier Publ. Amsterdam-New York-London 1973 bis 1979).

Die für die Durchführung der Erfindung geeigneten Sulfenhalogenide der allgemeinen Formel (II) sind bekannt oder können ebenfalls nach bekannten Methoden hergestellt werden (vgl. E. Kühle, The Chemistry of the Sulphenic Acids, G. Thieme Verlag, Stuttgart 1973, S. 2-37).

Beispiele erfindungsgemäßer Verbindungen sind:

N-(2-Pyrimidinyl)-phenylsulfenamid

N-(2-Pyrimidinyl)-4-chlorphenyl-sulfenamid
N-(2-Pyrimidinyl)-pentachlorphenyl-sulfenamid
N-(2-Pyrimidinyl)-4-(1,1-dimethylethyl)-phenyl-sulfenamid
N-(2-Pyrimidinyl)-1-naphthyl-sulfenamid
N-(2-Pyrimidinyl)-cyclohexyl-sulfenamid
N-(2-Pyrimidinyl)-isopropyl-sulfenamid
N-(1,2,4-Triazol-3-yl)-phenyl-sulfenamid
N-(1,2,4-Thiazol-3-yl)-4-chlorphenyl-sulfenamid
N-(1,2,4-Triazol-3-yl)-4-carbomethoxyphenyl-sulfenamid
N-(1,2,4-Triazol-3-yl)-4-(N,N-dimethylcarbamoyl)-phenyl-sulfenamid
N-(1,2,4-Triazol-3-yl)-3-carbomethoxyphenyl-sulfenamid
N-(1,2,4-Triazol-3-yl)-4-methylphenyl-sulfenamid
N-(1,2,4-Triazol-3-yl)-4-(1,1-dimethylethyl)-phenyl-sulfenamid
N-(1,2,4-Triazol-3-yl)-heptafluorpropyl-sulfenamid
N-(1,2,4-Triazol-3-yl)-benzyl-sulfenamid
N-(1,2,4-Triazol-3-yl)-cyclohexyl-sulfenamid
N-(1,2,4-Triazol-3-yl)-butylsulfenamid
N-(5-Methyl-1,2,4-triazol-3-yl)-phenyl-sulfenamid
N-(5-Methyl-1,2,4-triazol-3-yl)-4-chlorphenyl-sulfenamid
N-(5-Methyl-1,2,4-triazol-3-yl)-4-methylphenyl-sulfen-amid
N-(5-Methyl-1,2,4-triazol-3-yl)-4-(1,1-dimethylethyl)-phenyl-sulfenamid
N-(5-Methyl-1,2,4-triazol-3-yl)-cyclohexylsulfenamid
N-(5-Methyl-1,2,4-triazol-3-yl)-2-naphthyl-sulfenamid
N-(5-Phenyl-1,2,4-triazol-3-yl)-phenylsulfenamid
N-(5-Phenyl-1,2,4-triazol-3-yl)-4-chlorphenylsulfenamid

N-(5-Phenyl-1,2,4-triazol-3-yl)-4-methylphenylsulfenamid
N-(5-Phenyl-1,2,4-triazol-3-yl)-cyclohexylsulfenamid
N-(1,2,4-Thiadiazol-5-yl)-phenylsulfenamid
N-(1,2,4-Thiadiazol-5-yl)-4-chlorphenylsulfenamid
N-(1,2,4-Thiadiazol-5-yl)-4-methylphenylsulfenamid
N-(1,2,4-Thiadiazol-5-yl)-cyclohexylsulfenamid
N-(1,2,4-Thiadiazol-3-yl)-phenylsulfenamid
N-(1,2,4-Thiadiazol-3-yl)-4-chlorphenyl-sulfenamid
N-(1,2,4-Thiadiazol-3-yl)-cyclohexyl-sulfenamid
N-(3-Methyl-1,2,4-thiadiazol-5-yl)-phenyl-sulfenamid
N-(3-Methyl-1,2,4-thiadiazol-5-yl)-4-chlorphenyl-sulfenamid
N-(3-Methyl-1,2,4-thiadiazol-5-yl)-4-methylphenylsulfenamid
N-(3-Methyl-1,2,4-thiadiazol-5-yl)-cyclohexyl-sulfenamid
N-(5-Methyl-1,2,4-thiadiazol-3-yl)-cyclohexyl-sulfenamid
N-(5-Methyl-1,2,4-thiadiazol-3-yl)-phenyl-sulfenamid
N-(3-Methyl-1,2,4-thiadiazol-3-yl)-4-chlorphenyl-sulfenamid
N-(3-Methyl-1,2,4-thiadiazol-3-yl)-4-(1,1-dimethylethylphenyl)-sulfenamid
N-(3-Phenyl-1,2,4-thiadiazol-5-yl)-4-(1,1-dimethylethylphenyl)-sulfenamid
N-(3-Phenyl-1,2,4-thiadiazol-5-yl)-phenyl-sulfenamid
N-(3-Phenyl-1,2,4-thiadiazol-5-yl)-4-chlorphenylsulfenamid
N-(3-Phenyl-1,2,4-thiadiazol-5-yl)-cyclohexyl-sulfenamid
N-(3-Phenyl-1,2,4-thiadiazol-5-yl)-4-methylphenylsulfenamid

N-(3-Phenyl-1,2,4-thiadiazol-5-yl)-3-trifluormethylphenyl-sulfenamid
N-(5-Phenyl-1,2,4-thiadiazol-3-yl)-phenyl-sulfenamid
N-(5-Phenyl-1,2,4-thiadiazol-3-yl)-4-carbomethoxyphenyl-sulfenamid
N-(5-Phenyl-1,2,4-thiadiazol-3-yl)-cyclohexyl-sulfenamid
N-(3-Propyl-1,2,4-thiadiazol-5-yl)-cyclohexyl-sulfenamid
N-(3-Propyl-1,2,4-thiadiazol-5-yl)-phenyl-sulfenamid
N-(3-Propyl-1,2,4-thiadiazol-5-yl)-4-chlorphenyl-sulfenamid
N-(3-Propyl-1,2,4-thiadiazol-5-yl)-4-(1,1-dimethylethyl)-phenyl-sulfenamid
N-(1,3,4-Thiadiazol-2-yl)-phenyl-sulfenamid
N-(1,3,4-Thiadiazol-2-yl)-4-chlorphenyl-sulfenamid
N-(1,3,4-Thiadiazol-2-yl)-cyclohexyl-sulfenamid
N-(5-Dimethylamino-1,3,4-thiadiazol-2-yl)-phenyl-sulfenamid
N-(5-Dimethylamino-1,3,4-thiadiazol-2-yl)-4-chlorphenyl-sulfenamid
N-(5-Dimethylamino-1,3,4-thiadiazol-2-yl)-4-methoxyphenyl-sulfenamid
N-(5-Chlor-1,3,4-thiadiazol-2-yl)-phenyl-sulfenamid
N-(5-Chlor-1,3,4-thiadiazol-2-yl)-4-chlorphenyl-sulfenamid
N-(5-Chlor-1,3,4-thiadiazol-2-yl)-cyclohexyl-sulfenamid
N-(5-Methylmerkapto-1,3,4-thiadiazol-2-yl)-cyclohexyl-sulfenamid
N-(5-Methylmerkapto-1,3,4-thiadiazol-2-yl)-phenyl-sulfenamid

N-(5-Phenyl-1,3,4-thiadiazol-2-yl)-phenyl-sulfenamid
N-(5-Phenyl-1,3,4-thiadiazol-2-yl)-cyclohexyl-sulfenamid
N-(5-Methyl-1,3,4-thiadiazol-2-yl)-cyclohexyl-sulfenamid
N-(5-Methyl-1,3,4-thiadiazol-2-yl)-phenyl-sulfenamid
N-(5-Isopropyl-1,3,4-thiadiazol-2-yl)-phenyl-sulfenamid
N-(2-Pyridyl)-phenylsulfenamid
N-(2-Pyridyl)-4-chlorphenylsulfenamid
N-(2-Pyridyl)-4-(1,1-dimethylethyl)-phenylsulfenamid
N-(2-Pyridyl)-cyclohexyl-sulfenamid
N-(4,6-Dimethyl-2-pyrimidinyl)-phenyl-sulfenamid
N-(4,6-Diphenyl-2-pyrimidinyl)-phenyl-sulfenamid
N-(5-Chlor-2-pyrimidinyl)-phenyl-sulfenamid
N-(5-Tetrazolyl)-phenyl-sulfenamid
N-(5-Tetrazolyl)-cyclohexyl-sulfenamid
N-(5-Tetrazolyl)-4-isocyanatophenyl-sulfenamid
N-(5-Tetrazolyl)-dodecanyl-sulfenamid
N-(5-Tetrazolyl)-isopropyl-sulfenamid
N-(5-Tetrazolyl)-butyl-sulfenamid
N-(5-Tetrazolyl)-4-chlorphenyl-sulfenamid
N-(5-Tetrazolyl)-4-methylphenyl-sulfenamid
N-(1-Phenyl-4-phenylthio-5-pyrazolon-3-yl)-phenyl-sulfenamid
N-(1-Phenyl-4-cyclohexylthio-5-pyrazolon-3-yl)-cyclohexyl-sulfenamid

Der Nachweis der lipoxygenasehemmenden Eigenschaften der Sulfenamide erfolgt analog der Methode von Bailey et al., J. Biol. Chem. 255, 5996, (1980)

und nach Blackwell und Flower, Brostaglandins 16, 417 (1979). Bei dieser Testmethode wird der Metabolismus radioaktiv markierter Arachidonsäure an gewaschenen Humanthrombozyten herangezogen. Bei diesem in vitro Test werden die radioaktiv markierten Metaboliten aus dem Reaktionsansatz extrahiert und dünnschichtchromatographisch getrennt. Das Autoradiogramm wird am Dünnschicht-Scanner ausgewertet. Bei diesen Testbedingungen werden die markierten Metaboliten von der nicht umgesetzten Arachidonsäure getrennt und sind anschließend quantitativ auswertbar. Die Verteilung der Radioaktivität auf die während der Metabolisierung gebildeten Cyclooxygenaseprodukte Thromboxan $B_2$ ($TXB_2$) und 12-Hydroxy-5,8,10-heptadekatriensäure (HHT) bzw. das Lipoxygenaseprodukt 12-Hydroxy-5,8,11,14-eikosatetraensäure (HETE) unter dem Einfluß der Inhibitoren stellt ein Maß für die Hemmung der Enzyme dar.

Die Lipoxygenasehemmung der erfindungsgemäßen Sulfenamide läßt sich an der Hemmung der HETE-Synthese messen. Es zeigt sich, daß die Synthese von $TXB_2$ und von HHT unbeeinflußt bleibt, während der Arachidonsäureumsatz abnimmt. Wie aus der nachfolgenden Tabelle ersichtlich ist, bewirken die Sulfenamide eine signifikante Hemmung der Plättchenlipoxygenase (HETE-Synthese).

Hemmung der Plättchenlipoxygenase (HETE-Synthese)

| Verbindung aus Beispiel Nr. (siehe unten) | Minimale effektive Hemmkonzentration (g/ml) (mindestens 50 % Hemmung) |
|---|---|
| 2 | $10^{-6}$ |
| 4 | $10^{-6}$ |
| 5 | $10^{-6}$ |
| 6 | $10^{-6}$ |
| 7 | $10^{-6}$ |
| 8 | $10^{-6}$ |
| 10 | $3 \times 10^{-6}$ |
| 12 | $3 \times 10^{-6}$ |
| 13 | $10^{-6}$ |
| 16 | $3 \times 10^{-6}$ |
| 17 | $10^{-6}$ |
| 18 | $10^{-6}$ |
| 19 | $3 \times 10^{-6}$ |
| 20 | $3 \times 10^{-6}$ |
| 23 | $10^{-5}$ |
| 24 | $3 \times 10^{-6}$ |

Die erfindungsgemäßen Sulfenamide sind auch in vitro wirksam. Diese Wirkung wird durch die Messung der Hemmung der Leukozytenmigration nach an sich bekannten Methoden nachgewiesen (vgl. Higgs et al., Biochemical Pharmacology 28, 1959 (1979) und Eur. J. Pharmacol. 66, 81 (1980)). In der nachfolgenden Tabelle 2 sind die Wirkungen einiger beispielhafter Sulfenamide nach lokaler Applikation, durch Einführen eines mit Wirkstoff getränkten Schwämmchens unter die Rückenhaut von Ratten, zusammengefaßt.

| Verbindung aus Beispiel Nr. | Dosis, lokal (mg/Ratte) | Hemmung der Leukozytenmigration (Kontrolle = 0 %) |
|---|---|---|
| 2 | 10 | 45 % |
| 7 | 10 | 70 % |
| 9 | 10 | 61 % |
| 11 | 10 | 76 % |
| 12 | 10 | 35 % |
| 16 | 10 | 67 % |
| 17 | 10 | 53 % |
| 25 | 10 | 80 % |
| 28 | 10 | 29 % |
| 30 | 10 | 31 % |
| 37 | 10 | 22 % |
| 39 | 10 | 60 % |

Die Antiasthmatische Wirkung der erfindungsgemäßen Verbindung kann ebenfalls nach bereits bekannten Methoden nachgewiesen werden (vgl. Samuelson et al., FEBS Letters, 110, 213 (1980) und Yen et al., Agents and Actions 10, 274 (1980)).

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide),

synthetische Gesteinsmehle (z.B. hochdisperse Kieselsäure, Silikate), Zucker (z.B. Roh-, Milch- und Traubenzucker), Emulgiermittel, wie nicht-ionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Die folgenden Beispiele sollen die Erfindung näher erläutern:

Beispiel 1

Zur Suspension von 47,5 g (0,5 Mol) 2-Pyrimidinamin und 44 g Pyridin in 100 ml trockenem Dimethylformamid tropft man eine Lösung von 0,5 Mol 2-Propylsulfenchlorid in 300 ml Chlorbenzol. Man läßt 2 Stunden bei Raumtemperatur nachrühren, saugt vom ausgefallenen Pyridinhydrochlorid ab und schüttelt die Mutterlauge zweimal mit Eiswasser aus. Die organische Phase wird über Natriumsulfat getrocknet, das Lösungsmittel im Vakuum abgedampft und der Rückstand aus Leichtbenzin umkristallisiert. Man erhält 30 g N-2-Pyrimidin-isopropylsulfenamid als beigegelbe Kristalle vom Schmp. 78-84°C.

1H-NMR ($CDCl_3$): $\delta$= 8,51 (d,J = 4,5 Hz, 2H); 8,29 (s. breit, NH); 6,78 (t,J = 4,5 Hz, 1H); 3,33 (heptett, J= 7 Hz); 1,25 ppm (d,J = 7 Hz, 6H).

Beispiel 2-24

Analog Beispiel 1 wurden die folgenden Beispiele erhalten, siehe Tabelle 3. In einigen Fällen waren die erhaltenen Sulfenamide schwer löslich, so daß sie durch Abfiltrieren und Waschen mit Eiswasser isoliert werden konnten.

Tabelle 3

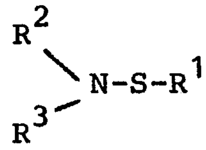

| Beispiel | $-NR^2R^3$ | $R^1$ | Verwendetes Lösungsmittel | Ausbeute (%) | Schmp. (°C) (Umkrist. aus) 1) | Bemerkungen |
|---|---|---|---|---|---|---|
| 2 | 2-Pyrimidinyl-NH- | Phenyl | Chlorbenzol/DMF | 33 | 122-7 (Me) | |
| 3 | " | Cyclo-hexyl | Pentan/DMF | 24 | 67-83 | |
| 4 | " | Penta-chlor-phenyl | Chlorbenzol/DMF | 83 | 208-10 | |
| 5 | " | 4-Chlor-phenyl | " | 13 | 155-7 (Cy/Tol) | |
| 6 | " | 4-tert.-Butyl-phenyl | Toluol/DMF | 12 | 139-41 (EE) | |
| 7 | 3-Amino-1,2,4-triazolyl | Phenyl | " | 61 | 154°C (EE) | |
| 8 | " | 4-Chlor-phenyl | " | 46 | 154-6°C (EE) | Bildet sich beim Umkrist. aus Bsp.10 |
| 9 | " | 4-Methyl-phenyl | " | 43 | 130-2°C | |
| 10 | 1,2,4-Triazol-3-yl-NH- | 4-Chlor-phenyl | " | 62 | | |
| 11 | " | Phenyl | " | | 163 (EE) | |

Le A 21 013

Tabelle 3 (Fortsetzung)

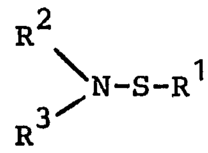

| Beispiel | $-NR^2R^3$ | $R^1$ | Verwendetes Lösungsmittel | Ausbeute (%) | Schmp. (°C) (Umkrist. aus) 1) | Bemerkungen |
|---|---|---|---|---|---|---|
| 12 | 3-Phenyl-1,2,4-thiadiazol-5-yl-NH- | Phenyl | Chlorbenzol/DMF | 57 | 154-7°C (Tol) | |
| 13 | " | 4-Chlorphenyl | Chlorbenzol/DMF | 61 | 156-7 (Cy/Tol) | |
| 14 | " | 4-Methylphenyl | Toluol/DMF | 44 | 123-5 (iPr) | |
| 15 | " | 4-tert.-Butylphenyl | " | 45 | 190-2 (EE) | |
| 16 | 3-Methyl-1,2,4-thiadiazol-5-yl-NH- | Phenyl | $CCl_4$ | 40 | 102-3 | |
| 17 | " | 4-Chlorphenyl | $CCl_4$ | 67 | 154-6 (Cy/Tol) | |
| 18 | 5-Methyl-1,2,4-thiadiazol-3-yl-NH- | Phenyl | Xylol | | | |
| 19 | 3-Propyl-1,2,4-thiadiazol-5-yl-NH- | Phenyl | Methylenchlorid/DMF | 32 | 85-6 (WB) | |
| 20 | " | 4-Chlorphenyl | Toluol/DMF | 55 | 154-5 (Cy/Tol) | |

Tabelle 3 (Fortsetzung)

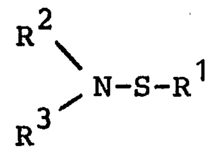

| Beispiel | $-NR^2R^3$ | $R^1$ | Verwendetes Lösungsmittel | Ausbeute (%) | Schmp. (°C) (Umkrist. aus) 1) | Bemerkungen |
|---|---|---|---|---|---|---|
| 21 | 5-Isopropyl-1,3,4-thiadiazol-2-yl-NH- | Phenyl | Chlorbenzol | 70 | 129-31 | |
| 22 | 4,6-Dimethyl-2-pyrimidinyl-NH- | Phenyl | Chlorbenzol/DMF | 53 | 161-4 (WB) | |
| 23 | 2-Pyridyl-NH- | 4-Chlorphenyl | Chlorbenzol/DMF | 66 | 105-7 | |
| 24 | 1-Phenyl-4-phenylthiopyrazol-5-on-3-yl-NH- | Phenyl | Toluol/DMF | 65 | 178-80 (EE) | |
| 25 | 5-Methyl-1,3,4-thiadiazol-2-yl-NH- | Phenyl | Toluol/DMF | 64 | 124-6 (Tol) | |
| 26 | 3-Methyl-1,2,4-thiadiazol-5-yl-NH- | 4-Chlor-3-Trifluormethylphenyl | Toluol/DMF | ca.30 | 123-7 | Mehrmals mit Petrolether gewaschen |
| 27 | " | 3-Trifluormethylphenyl | Toluol/DMF | 42 | 142-4 | |

Tabelle 3 (Fortsetzung)

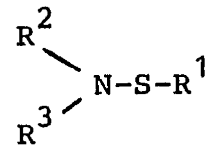

| Bei- spiel | $-NR^2R^3$ | $R^1$ | Verwendetes Lösungsmittel | Ausbeute (%) | Schmp. (°C) (Umkrist. aus) 1) | Bemerkungen |
|---|---|---|---|---|---|---|
| 28 | 1,2,4-Triazol-3-yl-NH- | 4-Chlor-3-Trifluormethylphenyl | Toluol/DMF | 75 | 171-3 | Von Zugabe von Leichtbenzin gefällt |
| 29 | " | 3-Trifluormethylphenyl | Chlorbenzol/DMF | 38 | 152-3 | |
| 30 | " | 4-Carbomethoxyphenyl | Toluol/DMF | 33 | 170 (Zers.) | Mit heißem Toluol gewaschen |
| 31 | " | 2-Chlor-5-Trifluormethylphenyl | Toluol/DMF | 43 | 193 (Zers.) | |
| 32 | " | 2-Nitrophenyl | Toluol/DMF | 29 | 209-211 (Dioxan/Tol) | |
| 33 | " | 4-Fluorphenyl | Toluol/DMF | 41 | 166-7 (viel EE) | |
| 34 | 3-Methyl-1,2,4-thiadiazol-5-yl-NH- | 2-Nitrophenyl | Toluol/DMF | 38 | 201-3 (viel Tol) | |

Tabelle 3 (Fortsetzung)

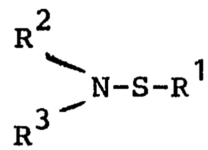

| Beispiel | $-NR^2R^3$ | $R^1$ | Verwendetes Lösungsmittel | Ausbeute (%) | Schmp. (°C) (Umkrist. aus) 1) | Bemerkungen |
|---|---|---|---|---|---|---|
| 35 | 5-Methyl-1,3,4-thiadiazol-2-yl-NH- | 4-Fluorphenyl | Toluol/DMF | 50 | 132-4 (Cy/Tol) | |
| 36 | 6-Carbomethoxy-1,3-thiazin-4-on-2-yl-NH- | 4-Chlorphenyl | Toluol/DMF | 98 | 165-8 | |
| 37 | 5-(Morpholin-4-yl)-1,3,4-thiadiazol-2-yl-NH- | " | Toluol/DMF | 94 | 143-5 | |
| 38 | 3-Phenyl-1,2,4-thiadiazol-yl-NH- | 2-Carbomethoxyphenyl | Toluol/DMF | 39 | 195-200° (Zers.) (Cy/Tol.) | |

Tabelle 3 (Fortsetzung)

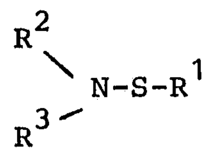

| Bei-spiel | $-NR^2R^3$ | $R^1$ | Verwendetes Lösungsmittel | Ausbeute (%) | Schmp. (°C) (Umkrist. aus) 1) | Bemerkungen |
|---|---|---|---|---|---|---|
| 39 | 5-Methyl-1,3,4-thia-diazol-2-yl-NH- | 4-Chlor-phenyl | Chlorbenzol/DMF | 65 | 150 (Zers.) (Tol) | |
| 40 | 5-Trifluor-methyl-1,3,4-diaziazol-2-yl-NH- | Phenyl | Toluol/DMF | 30 | | |

Erläuterungen zu Tabelle 3

Cy = Cyclohexan
EE = Essigsäureethylester
iPr = i-Propanol
Me = Methanol
Tol = Toluol
WB = Waschbenzin (Kp. 100-140°C)

Beispiele 41-42

42,5 g (0,5 Mol) 5-Aminotetrazol werden in 100 ml Wasser und 20 g (0,5 Mol) Natronlauge gelöst. Man gibt 100 ml Chlorbenzol zu und tropft dann bei +10°C eine Lösung aus 0,5 Mol Cyclohexylsulfenchlorid in 400 ml Chlorbenzol langsam zu. Nach beendeter Zugabe läßt man 30 Minuten nachrühren und nutscht dann ab. Der Rückstand wird mit Petrolether gewaschen und dann über $P_4O_{10}$ getrocknet. Man erhält 66 % der Theorie an N-5-Tetrazolyl-cyclohexylsulfenamid mit Schmp. 125-7°C (Zers.).

Analog erhält man mit 0,5 Mol n-Dodecylsulfenchlorid 74,5 % der Theorie N-5-Tetrazolyl-dodecylsulfenamid mit Schmp. 161°C (Zers.).

Patentansprüche

1. Sulfenamide der allgemeinen Formel I

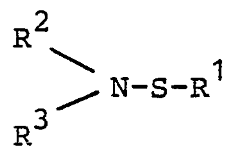

in welcher

$R^1$ für Alkyl mit 1 bis 18 C-Atomen, Alkenyl oder Alkinyl mit 2 bis 12 C-Atomen, Aralkyl mit 7 bis 11 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkoxy, Alkyl, Aralkyl, Cycloalkyl, Aryl, Aryloxy, Arylthio, Alkylthio, Carboxy, Carbalkoxy, Cyano, Carbamoyl, Sulfonyl, Halogenalkyl, Halogenalkoxy, Halogen, Amino oder substituiertes Amino substituiert sind, oder $R^1$ für den Rest

$$-Q-S-NR^2R^{3'} \qquad \text{(Ia)}$$

steht,
wobei
$R^2$ die unten angegebene Bedeutung hat und
$R^{3'}$ die unter $R^3$ angegebene Bedeutung hat außer einem Rest der Formel (Ia),
Q für Alkylen mit 1 bis 12 C-Atomen, wobei der Alkylenrest durch Sauerstoff, Schwefel oder Stickstoff ein- oder mehrfach unterbrochen sein kann, Cycloalkylen mit mit 5 bis 12 C-Atomen, Arylen mit 6 bis 10 C-Atomen, Alkylen-Cycloalkylen-Alkylen oder Alkylen-Arylen-Alkylen steht, mit der Einschränkung, daß $R^1$ nicht für Trichlormethyl steht,

$R^2$ einen heterocyclischen Rest mit 5 bis 8 Ringgliedern, welcher 1 bis 4 Heteroatome aus der Gruppe Sauerstoff, Schwefel oder Stickstoff enthält, wobei dieser heterocyclische Rest gegebenenfalls mit einem Arylrest annelliert ist und bis zu 5 gleiche oder verschiedene Substituenten aus der Gruppe $R^{1'}$, worin

$R^{1'}$ einer der Reste der Definition von $R^1$, jedoch nicht den Rest der Formel (Ia), bedeutet;

Halogen, Alkylthio, Cycloalkylthio, Aralkylthio, Arylthio, Alkoxy, Aryloxy, Cyano, Nitro, Cycloalkoxy, Aralkoxy oder $-CO-R^4$,

worin

$R^4$ für Wasserstoff, Alkyl, Cycloalkyl, Alkenyl, Alkinyl, Aralkyl, Aryl, Alkoxy, Aralkoxy, Aryloxy, Alkylthio, Arylthio oder $-NR^{3''}R^5$ steht,

worin

$R^{3''}$ die unter $R^{3'}$ angegebene Bedeutung hat, jedoch nicht den Rest $R^2$ und

$R^5$ Wasserstoff oder einen der unter $R^{1'}$ genannten Reste bedeutet,

oder einen Rest der allgemeinen Formel

$$-W-CO-NR^{3''}R^{5'} \quad (Ib)$$

bedeutet,

wobei

W für eine direkte Bindung, eine Disulfidbrücke oder für eines der oben unter Q genannten Brückenglieder steht, und

$R^{5'}$ die in der Definition für $R^5$ außer dem Rest der Formel (Ib) angegebenen Bedeutung besitzt, oder

$SO_2-R^6$, worin

$R^6$ für Fluoralkyl, einen der unter $R^4$ aufgeführten Reste oder für

$$-W-SO_2-NR^{3''}R^5 \quad (Ic)$$

steht, wobei

$R^{3''}$, $R^5$ und W die oben angegebene Bedeutung haben,

oder

$-NR^{3''}R^5$, Trifluormethyl, Trifluormethoxy, Oxo, Thiono, Imino oder substituiertes Imino enthalten kann, wobei als Substituenten der Iminogruppe die oben unter $R^1$ genannten Reste oder ein Carbamoylrest, der am Stickstoff einen Substituenten aus der Gruppe Alkyl, Aryl oder Cycloalkyl trägt, genannt seien, mit der Einschränkung, daß $R^2$ nicht für 2-Oxazolyl, 2-Benzoxazolyl, 2-Thiazolyl, 2-Benzothiazolyl und 2-Benzimidazolyl steht,

und

$R^3$ für einen der unter $R^1$ oder $R^2$ aufgezählten Reste steht oder Wasserstoff oder $-S-R^1$ bedeutet.

0065226

2. Sulfenamide der allgemeinen Formel (I) Anspruch 1, in welcher

$R^1$ für Alkyl mit 1 bis 18 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl mit 5 bis 7 Kohlenstoffatomen, Phenyl, Phenoxy, Phenylthio, Alkylthio mit 1 bis 4 Kohlenstoffatomen, Carboxy, Carbalkoxy mit 1 bis 4 Kohlenstoffatomen, Cyano, Trifluormethoxy, Fluor, Chlor, Brom, Amino, Alkyl- oder Benzyl-substituiertes Amino, wobei die Alkylreste 1 bis 4 Kohlenstoffatomen tragen, substituiert sind, mit der Einschränkung, daß $R^1$ nicht für Trichlormethyl steht,

$R^2$ für einen heterocyclischen Rest mit 5 bis 8 Ringgliedern steht, welcher 1 bis 4 Heteroatome aus der Gruppe Sauerstoff, Schwefel oder Stickstoff enthält, wobei dieser heterocyclische Rest gegebenenfalls mit einem Arylrest mit 6 bis 14 C-Atomen annelliert ist und bis zu 5 gleiche oder verschiedene Substituenten aus der Gruppe Fluor, Chlor, Brom, Alkylthio mit 1 bis 4 C-Atomen, Benzylthio, Phenylthio, Alkoxy mit 1 bis 4 C-Atomen, Phenoxy, Benzyloxy, einen der unter $R^1$ genannten Reste, $-NR^{3"}R^5$, $-CO-R^4$, Trifluormethyl, Trifluormethoxy, Oxo oder Thiono enthalten kann, mit der Einschränkung, daß $R^2$ nicht für 2-

Benzoxazolyl, 2-Thiazolyl, 2-Benzothiazolyl und 2-Benzimidazolyl steht, und

$R^3$ für einen der unter $R^1$ oder $R^2$ aufgezählten Reste steht oder Wasserstoff oder $-S-R^1$ bedeutet, wobei $R^1$ und $R^2$ die angegebene bevorzugte Bedeutung besitzen.

3. Sulfenamide der allgemeinen Formel (I) Anspruch 1, in welcher

$R^1$ für einen Arylrest mit 6 bis 14 C-Atomen steht, der gegebenenfalls mit bis zu 2 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl mit 5 bis 7 Kohlenstoffatomen, Phenyl, Phenoxy, Phenylthio, Alkylthio mit 1 bis 4 Kohlenstoffatomen, Carbalkoxy mit 1 bis 4 Kohlenstoffatomen, Cyano, Trifluormethyl, Trifluormethoxy, Fluor, Chlor, Brom, Alkyl- oder Benzyl-substituiertes Amino, wobei die Alkylreste 1 bis 4 Kohlenstoffatome tragen, substituiert ist,

$R^2$ für einen heterocyclischen Rest mit 5 oder 6 Ringgliedern, welcher 1 bis 4 Heteroatome aus der Gruppe Sauerstoff, Schwefel oder Stickstoff enthält, wobei dieser heterocyclische Rest gegebenenfalls benzannelliert ist und bis zu 3 gleiche oder verschiedene Substituenten aus der Gruppe Fluor, Chlor, Brom, Alkylthio mit 1 bis 4 C-Atomen, Benzylthio, Phenylthio, Alkoxy mit 1 bis 4 C-Atomen, Phenoxy, Benzyloxy, Alkyl mit 1 bis 12 C-Atomen, Benzyl, Phenyl, $-NR^{3"}R^5$, $-CO-R^4$, Trifluormethyl, Trifluormethoxy, Oxo oder Thiono enthalten kann, mit der Einschränkung, daß $R^2$ nicht für 2-Oxazolyl, 2-Benzoxazolyl, 2-Thiazolyl, 2-Benzothiazolyl und 2-Benzimidazolyl steht, und

$R^3$ für Wasserstoff oder Methyl steht.

4. Verfahren zur Herstellung von Sulfenamiden der allgemeinen Formel I gemäß Anspruch 1, dadurch gekennzeichnet, daß man ein Sulfenylhalogenid der allgemeinen Formel II

$$R^1\text{-S-X} \qquad \text{(II)}$$

in welcher

X für Chlor, Brom oder Jod steht und

$R^1$ die oben angegebene Bedeutung hat,

mit Aminen der allgemeinen Formel III

$$Y\text{-NR}^2R^3 \qquad \text{(III)}$$

in welcher

Y für Wasserstoff, Trialkylsilyl, ein Metall wie K, Na, Li, Mg, Ca, Ba, Ag, Cu, Zn, Fe, Mn, Pb, Sn oder Al oder einen Ammoniumrest steht und

$R^2$ und $R^3$ die oben angegebene Bedeutung haben,

in Gegenwart von inerten aprotischen organischen Lösungsmitteln und gegebenenfalls in Gegenwart von Wasser und von Basen bei Temperaturen zwischen -80 und 150°C unter Abspaltung von XY umsetzt.

5. Verwendung von Sulfenamiden der allgemeinen Formel I gemäß Anspruch 1 als Arzneimittel.

6. Verwendung von Sulfenamiden gemäß Anspruch 1 bis 3 als Lipoxygenasehemmer.

7. Verwendung von Sulfenamiden gemäß Anspruch 1 bis 3 als Antiphlogistika, Antirheumatika, Antiatherosklerotika, Antiasthmatika, Antiallergika, Antimetastatika und Gastroprotektiva.

8. Arzneimittel enthaltend mindestens eine Verbindung der allgemeinen Formel I gemäß Anspruch 1.

9. Arzneimittel mit Lipoxygenase-hemmender Wirkung enthaltend mindestens eine Verbindung der allgemeinen Formel I gemäß Anspruch 1.

10. Verfahren zur Herstellung von Arzneimitteln, dadurch gekennzeichnet, daß man Sulfenamide der allgemeinen Formel I gemäß Anspruch 1, gegebenenfalls zusammen mit Hilfs- und Trägerstoffen in eine geeignete Applikationsform überführt.